# NEUE EUROPÄISCHE PATENTSCHRIFT

(11) **EP 0 815 141 B2**
(45) Veröffentlichungstag und Bekanntmachung des Hinweises auf die Entscheidung über den Einspruch: **25.02.2009**
(45) Hinweis auf die Patenterteilung: 31.10.2001
(21) Anmeldenummer: 96904728.1
(22) Anmeldetag: 01.03.1996
(51) Int. Cl.: C07K 16/00, G01N 33/53

(54) **ANTIKÖRPER GEGEN EIN, EINEN HISTIDIN-ANTEIL AUFWEISENDES FUSIONSPOLYPEPTID**
ANTIBODIES ACTIVE AGAINST A FUSION POLYPEPTIDE COMPRISING A HISTIDINE PORTION
ANTICORPS AGISSANT A L'ENCONTRE D'UN POLYPEPTIDE FUSIONNE COMPORTANT UNE PARTIE HISTIDINE

(30) Priorität: 01.03.1995 DE 19507166
(43) Veröffentlichungstag der Anmeldung: 07.01.1998
(73) Patentinhaber: Deutsches Krebsforschungszentrum Stiftung des öffentlichen Rechts, 69120 Heidelberg (DE)
(72) Erfinder: ZENTGRAF, Hanswalter, D-69115 Heidelberg (DE); TESSMER, Claudia, D-74869 Schwarzach (DE); VELHAGEN, Iris, D-68723 Schwetzingen (DE); SCHWINN, Susanne, D-68766 Hockenheim (DE); FREY, Manfred, D-68259 Mannheim (DE)
(74) Vertreter: Schüssler, Andrea
(86) Internationale Anmeldenummer: PCT/DE1996/000369
(87) Internationale Veröffentlichungsnummer: WO 1996/026963

(56) Entgegenhaltungen:
- WO-A-94/08241
- DE-A- 4 339 533
- DE-C- 19 507 166
- PROCEEDINGS OF THE NATIONAL ACADEMY OF SCIENCES,USA, Bd. 90, Oktober 1993, Seiten 9350-9354, XP002004587 CAMPBELL ET AL: "FUNCTIONAL COMPLEMENTATION OF AN ESCHERICHIA COLI RIBONUCLEASE H MUTATION BY A CLONED GENOMIC FRAGMENT FROM THE TRYPANOSOMATID CRITHIDIA FASCICULATA"
- VIROLOGY, Bd. 202, 1994, Seiten 1070-1075, XP002004588 PEDERSEN ET AL: "MOLECULAR CHARACTERIZATION OF THE AL3 PROTEIN ENCODED BY A BIPARTITE GEMINIVIRUS"
- VIROLOGY, Bd. 206, Nr. 1, 10.Januar 1995, Seiten 465-478, XP002004589 PATEL ET AL: "THE PRODUCT OF THE UL6 GENE OF HERPES SIMPLEX VIRUS TYPE 1 IS ASSOCIATED WITH VIRUS CAPSIDS"
- JOURNAL OF IMMUNOLOGICAL METHODS, Bd. 156, 1992, Seiten 231-238, XP002004590 EVANS ET AL: "IMMUNODETECTION OF RECOMBINANT PROTEINS BASED ON ANTIBODIES DIRECTED AGAINST A METAL BINDING PEPTIDE ENGINEERED FOR PURIFICATION BY IMMOBILIZED METAL AFFINITY CHROMATOGRAPHY"
- NUCLEIC ACIDS RESEARCH, Bd. 23, Nr. 16, 25.August 1995, Seiten 3347-3348, XP002004591 ZENTGRAF ET AL: "DETECTION OF HISTIDINE-TAGGED FUSION PROTEINS BY USING A HIGH-SPECIFIC MOUSE MONOCLONAL ANTI-HISTIDINE TAG ANTIBODY"

## Beschreibung

Die vorliegende Erfindung betrifft ein Verfahren zur Herstellung eines Antikörpers gegen ein, einen Histidin-Anteil aufweisendes Fusionspolypeptid.

Es ist bekannt, ein Polypeptid in Form eines Histidin-Fusionspolypeptids zu exprimieren. In einem solchen liegt ein Histidin-Anteil von z.B. 6-18 aufeinander folgenden Histidinresten fusioniert am C- oder N-Terminus des Polypeptids vor. Damit ist es möglich, das Histidin-Fusionspolypeptid mittels einer Nickel-Chelat-Chromatographiesäule aus dem Überstand oder Zellysat der es exprimierenden Zelle zu isolieren.

Vorstehende Säule ist aber teuer. Ferner bedeutet ihr Einsatz einen großen Zeitaufwand. Daher eignet sie sich nicht zum schnellen Nachweis der Expression eines Histidin-Fusionspolypeptids. Ein solcher Nachweis ist aber von Nöten, insbesondere, wenn er zum Screening vieler Zellen herangezogen werden soll.

Der vorliegenden Erfindung liegt somit die Aufgabe zugrunde, ein Mittel bereitzustellen, mit dem die Expression eines Histidin-Fusionspolypeptids schnell nachgewiesen werden kann.

Erfindungsgemäß wird dies durch ein Verfahren zur Herstellung eines Antikörpers erreicht, der gegen ein, einen Histidin-Anteil aufweisendes Fusionspolypeptid gerichtet ist, wobei der Antikörper gegen den Histidin-Anteil gerichtet ist und dieser 6-18 aufeinander folgende Histidinreste umfaßt. Ein solcher Antikörper kann ein polyklonaler oder monoklonaler Antikörper sein, wobei ein monoklonaler Antikörper bevorzugt ist. Der Antikörper kann aus jeglichem Tier oder dem Menschen erhalten sein, wobei für einen polyklonalen Antikörper Kaninchen und für einen monoklonalen Mäuse bevorzugt sind.

Ferner kann der Antikörper synthetisch sein, wobei ihm ggfs. Teile, die für vorstehende Erkennung nicht notwendig sind, ganz oder teilweise fehlen bzw. diese Teile durch andere ersetzt sind, die dem Antikörper weitere günstige Eigenschaften verleihen.

Der Ausdruck "Histidin-Anteil aufweisendes Fusionspolypeptid" umfaßt ein Polypeptid (Peptid) jeglicher Art und Länge, das einen Histidin-Anteil aufweist. Ein solches Polypeptid kann von jeglichen Zellen, z.B. Bakterien, Hefen, Insekten-, Pflanzen- und tierischen Zellen, sowie Organismen, z.B. transgenen Tieren, exprimiert sein. Ein vorstehender Histidin-Anteil umfaßt 6-18, vorzugsweise 6 aufeinander folgende Histidinreste und kann fusioniert am N und/oder C-Terminus des Polypeptids vorliegen.

Ein erfindungsgemäß hergestellter Antikörper, nämlich ein monoklonaler Maus-Antikörper mit vorstehender Erkennung, wurde bei der DSM unter der Nummer ACC 2207 am 15. Febr. 1995 hinterlegt.

Sollen polyklonale bzw. monoklonale Antikörper hergestellt werden, ist es günstig, Tiere, insbesondere Kaninchen für erstere und Mäuse für letztere Antikörper, mit einem Gemisch vorstehender Histidin-Fusionspolypeptide, z.B. His ⁻p53 (vgl. deutsche Patentanmeldung P 42 32 823.3) oder His hdm2 (vgl. deutsche Patentanmeldung P 43 39 553.3), zu immunisieren. Weiteres Boostern der Tiere kann mit dem oder den gleichen Histidin-Fusionspolypeptiden erfolgen. Auch können andere Histidin-Fusionspolypeptide oder eine Kombination aus diesen und dem oder den vorhergehenden Histidin-Fusionspolypeptiden zum Boostern verwendet werden. Die polyklonalen Antikörper können dann aus dem Serum der Tiere erhalten werden. Für die monoklonalen Antikörper werden Milzzellen der Tiere mit Myelomzellen fusioniert. Zur Herstellung von synthetischen Antikörpern kann z.B. von vorstehend erhaltenen, monoklonalen Antikörpern ausgegangen werden. Hierzu bietet sich an, die Antigen-Bindungsregionen der monoklonalen Antikörper zu analysieren und die für vorstehende Erkennung notwendigen und nicht notwendigen Teile zu identifizieren.
Die notwendigen Teile können dann modifiziert und die nicht notwendigen ganz oder teilweise eliminiert bzw. durch Teile ersetzt werden, die den Antikörpern weitere günstige Eigenschaften verleihen. Auch können Teile außerhalb der Bindungsregionen der Antikörper modifiziert, eliminiert oder ersetzt werden. Der Fachmann weiß, daß sich für vorstehende Maßnahmen insbesondere die DNA-Rekombinationstechnologie eignet. Diese ist ihm bestens vertraut.

Erfindungsgemäß hergestellte Antikörper zeichnen sich dadurch aus, daß sie beliebige Fusionspolypeptide erkennen, die einen Histidin-Anteil aufweisen. Die Antikörper eignen sich daher zum schnellen Nachweis der Expression solcher Fusionspolypeptide. Dies kann in beliebigen Nachweisverfahren, insbesondere in einem Western Blot, einem ELISA, einer Immunpräzipitation oder einer Immunfluoreszenz, erfolgen. Hierzu können die erfindungsgemäß hergestellten Antikörper, wenn es angebracht ist, markiert sein oder in Kombination mit markierten gegen sie gerichteten Antikörpern eingesetzt werden.

Die erfindungsgemäß hergestellten Antikörper zeichnen sich gegenüber den nachfolgend beschriebenen Antikörpern des Standes der Technik insbesondere dadurch aus, daß sie gegen den Histidin-Anteil eines Fusionspolypeptids gerichtet sind und nicht gegen einen der anderen Anteile des Fusionspolypeptids. So beschreiben Campbell et al., Proc. Natl. Acad. Sci. USA 90 (1993), S. 9350-9354 einen Antikörper gegen ein RNase H-Fusionsprotein; Pedersen et al., Virology 202 (1994), S. 1070-1075 beschreiben einen Antikörper gegen ein AL3-Fusionsprotein; Patel et al., Virology 206 (1995), S. 465-478 beschreiben einen Antikörper gegen ein UL6-Fusionsprotein und Evans et al., J. of Immunological Methods 156 (1992), S. 231-238 beschreiben einen Antikörper gegen ein Fusionsprotein, wobei der Antikörper den mbp-Anteil des Fusionsproteins erkennt.

Die vorliegende Erfindung wird durch die nachstehenden Beispiele erläutert.

### Beispiel 1: Herstellung von monoklonalen Antikörpern

Zur Immunisierung wurden Mäuse verwendet. Als Antigene wurden His hdm2 (Aminosäure 1-284), His hdm2 (Aminosäure 58-491) und His p53 (Aminosäure 66-393) (vgl. vorstehend) verwendet. Diese waren in einem Puffer aus 8 M Harnstoff, 100 mM NaH₂PO₄, 10 mM Tris-Hcl gelöst.

### Immunisierungs- und Boosterschema:

- Tag 1:: 50 *µ*l (= 10 *µ*g) His hdm2 (Aminosäure 1-284)
50 *µ*l (= 10 *µ*g) His hdm2 (Aminosäure 58-491)
50 *µ*l PBS (Phosphat-gepufferte Saline)
150 *µ*l Freund's Adjuvans komplett
-------
300 *µ*l Mix
200 *µ*l des Mix wurden in eine Maus injiziert
- Tag 30:: 50 *µ*l (= 10 *µ*g) His hdm2 (Aminosäure 1-284)
50 *µ*l (=10 µg) His hdm2 (Aminosäure 58-491)
20 *µ*l PBS
120 *µ*l Freund's Adjuvans inkomplett
-------
240 *µ*l Mix
200 *µ*l des Mix wurden in vorstehende Maus injiziert.
- Tag 60:: 50 *µ*l (= 10 *µ*g) His hdm2 (Aminosäure 1-284)
50 *µ*l (= 10 *µ*g) His hdm2 (Aminosäure 58-491)
85 *µ*l PBS
115 *µ*l Freund's Adjuvans inkomplett
--------
300 *µ*l Mix
200 *µ*l des Mix wurden in vorstehende Maus injiziert.
- Tag 90:: 50 *µ*l (= 10 *µ*g) His hdm2 (Aminosäure 1-284)
50 *µ*l (= 10 *µ*g) His hdm2 (Aminosäure 58-491)
200 *µ*l PBS
--------
300 *µ*l Mix
200 *µ*l des Mix wurden in vorstehende Maus injiziert.
- Tag 180:: 150 *µ*l (= 20 *µ*g) His p53 (Aminosäure 66-393)
150 *µ*l Freund's Adjuvans komplett
--------
300 *µ*l Mix
200 *µ*l des Mix wurden in vorstehende Maus injiziert.
- Tag 230:: 75 *µ*l (= 10 *µ*g) His p53 (Aminosäure 66-393)
25 *µ*l (= 5 *µ*g) His hdm2 (Aminosäure 1-284)
25 *µ*l (= 5 *µ*g) His hdm2 (Aminosäure 58-491)
125 *µ*l Freund's Adjuvans inkomplett
--------
250 *µ*l Mix
200 *µ*l des Mix wurden in vorstehende Maus injiziert.
- Tag 260:: 75 *µ*l (=10 *µ*g) His p53 (Aminosäure 66-393)
25 *µ*l (= 5 *µ*g) His hdm 2 (Aminosäure 1-284)
25 *µ*l (= 5 *µ*g) His hdm 2 (Aminosäure 58-491)
125 *µ*l PBS
--------
250 *µ*l Mix
200 *µ*l des Mix wurden in vorstehende Maus injiziert.

Am Tag 262 wurde die Maus getötet. Milzzellen wurden ihr entnommen und mit Myelomzellen fusioniert. Es wurden monoklonale Antikörper erhalten. Einer dieser wurde bei der DSM unter ACC 2207 am 15. Febr. 1995 hinterlegt.

### Beispiel 2: Herstellung von polyklonalen Antikörpern

Zur Immunisierung wurden Kaninchen verwendet. Es wurden die Antigene von Beispiel 1 verwendet. Das Immunisierungs- und Boosterschema war identisch mit jenem von Beispiel 1 bis einschließlich Tag 90.
- Tag 92:: Aus der Ohrvene des Kaninchens wurden 5 ml Blut entnommen und in einem ELISA bzw. Western-Blot auf Antikörper-Aktivität getestet.
- Tag 93:: Nach positivem Test am Tag 92 wurden die Tiere getötet und die Antikörper aus dem Serum gewonnen.

### Beispiel 3: Nachweis von Histidin-Fusionspolypeptiden durch erfindungsgemäß hergestellte Antikörper

### (a) Western-Blot

Histidin-Fusionspolypeptide, nämlich His hdm2 (Aminosäure 1-284), His hdm2 (Aminosäure 58-491) und His p53 (Aminosäure 66-393) von Beispiel 1, sowie die Polypeptide hdm2 (Aminosäure 1-284), WAF 1 (=wildtyp aktivierender Faktor) und t16 (= zell. regulierendes Protein) als Kontrolle wurden einer Polyacrylamid-Gelelektrophorese unterzogen. Das Gel wurde über Nacht auf eine Nitrocellulosemembran übertragen. Diese wurde dann mit vorstehendem, 1:10 bzw. 1:50 verdünnten Antikörper ACC 2207 1 Std. bei 37°C inkubiert. Nach mehreren Waschschritten mit PBS (0,05 % Tween 20) wurde ein käuflicher alkalischer Phosphatase-gekoppelter Ziege-anti-Maus-Antikörper (Verdünnung nach Angabe der Hersteller) zugegeben. Nach 30-minütiger Inkubation bei 37°C folgten mehrere Waschschritte mit PBS und anschließend die alkalische Phosphatase-Nachweisreaktion mit alkalischer Phosphatase mit Entwicklerlösung (36 *µ*M 5'Bromo-4-chloro-3-indolylphosphat, 400 *µ* M Nitroblau-tetrazolium, 100 mM Tris-HCl, pH 9.5, 100 mM NaCl, 5 mM MgCl₂) bei Raumtemperatur bis Banden sichtbar waren.

Es zeigte sich, daß der erfindungsgemäß hergestellte Antikörper ACC 2207 spezifisch Histidin-Fusionspolypeptide, nicht aber Polypeptide ohne Histidin-Anteil erkennt.

### (b) ELISA

In eine 96-Loch-Platte wurden pro Loch je 100*µ* l mit 20 ng bzw. 8 ng der Histidin-Fusionspolypeptide bzw. der Kontrollen von (a) einpipettiert. Nach Inkubation über Nacht bei 4°C schlossen sich 3 kurze Waschschritte mit PBS an. Anschließend erfolgte die Blockierung freier Bindungsstellen des polymeren Trägers durch einstündige Inkubation mit 1 % BSA in PBS bei 37°C. Der erfindungsgemäß hergestellte 1:10 bzw. 1:50 verdünnte Antikörper ACC 2207 wurde 1 Stunde bei 37°C auf der Platte inkubiert. Nach 8 Waschschritten mit PBS wurde der Peroxidase-gekoppelte Ziege anti-Maus Antikörper von (a) zugegeben. Nach 30-minütiger Inkubation bei 37°C folgten 8 Waschschritte und anschließend die Peroxidase-Nachweisreaktion mit Entwicklungslösung (50 mM Natriumacetat, 0,4 mM 3,3', 5,5'-Tetramethylbenzidin-dihydrochlorid, 4,4 mM H₂O₂) bei Raumtemperatur bis Banden sichtbar waren.

Es zeigte sich, daß der erfindungsgemäß hergestellte Antikörper ACC 2207 spezifisch Histidin-Fusionspolypeptide, nicht aber ein Polypeptid ohne Histidin-Anteil erkennt.

## Patentansprüche

1. Verfahren zur Herstellung eines Antikörpers gegen ein, einen Histidin-Anteil aufweisendes Fusionspolypeptid, wobei der Antikörper gegen den Histidin-Anteil gerichtet ist und dieser 6-18 aufeinander folgende Histidinreste umfaßt, **dadurch gekennzeichnet, daß** ein Tier mit einem Gemisch von Histidin-Fusionspolypeptiden immunisiert wird, und
(a)polyklonale Antikörper aus dem Serum des Tieres erhalten werden, oder
(b)monoklonale Antikörper nach Fusion von Milzzellen des Tieres mit Myelomzellen erhalten werden.

## Claims

1. Process for the preparation of an antibody against a fusion polypeptide comprising a histidine portion, wherein the antibody is directed against the histidine portion and the latter comprises 6-18 consecutive histidine residues, **characterized in that** an animal is immunized with a mixture of histidine fusion polypeptides and
(a) polyclonal antibodies are obtained from the serum of the animal, or
(b) monoclonal antibodies are obtained after the fusion of animal's spleen cells with myeloma cells.

## Revendications

1. Procédé pour préparer un anticorps agissant à l'encontre d'un polypeptide fusionné comportant une partie histidine, l'anticorps étant dirigé à l'encontre de la partie histidine et celle-ci comprenant de 6 à 18 restes d'histidine successifs, **caractérisé par le fait qu'**un animal est immunisé par un mélange de polypeptides fusionnés contenant de l'histidine, et
(a) des anticorps polyclonaux étant obtenus à partir du sérum de l'animal, ou
(b) des anticorps monoclonaux étant obtenus après fusion de cellule de rate de l'animal avec des cellule de myélome.
